# EUROPEAN PATENT SPECIFICATION

(11) **EP 0 973 948 B1**
(45) Date of publication and mention of the grant of the patent: **02.01.2008**
(21) Application number: 98912448.2
(22) Date of filing: 25.02.1998
(51) Int. Cl.: C12Q 1/70

(54) **NUCLEIC ACID BASED DETECTION OF CYTOMEGALOVIRUS**
CYTOMEGALOVIRUS NACHWEIS MIT NUKLEIN-SÄUREN
OLIGONUCLEOTIDES POUVANT ETRE UTILISES DANS L'AMPLIFICATION ET LA DETECTION DE L'ACIDE NUCLEIQUE DE CYTOMEGALOVIRUS (CMV)

(30) Priority: 28.02.1997 EP 97200623
(43) Date of publication of application: 26.01.2000
(73) Proprietor: bioMerieux B.V., 5280 AB Boxtel (NL)
(72) Inventor: SILLEKENS, Peter, Theodorus, Gerardus, NL-5291 ND Gemonde (NL); TIMMERMANS, Eveline, Catharina, Anna, Clasina, NL-5233 SR Gemonde (NL)
(74) Representative: Van Someren, Petronella F. H. M.
(86) International application number: PCT/EP1998/001280
(87) International publication number: WO 1998/038339

(56) References cited:
- WO-A-96/06191
- DEGRAAF M E ET AL: "BIOCHEMICAL DIVERSITY IN A PHAGE DISPLAY LIBRARY OF RANDOM DECAPEPTIDES" GENE, vol. 128, 1993, pages 13-17, XP002061371
- CHOU: "Effect of interstrain variation on diagnostic DNA amplification of the CMV major immediate early gene region" JOURNAL OF CLINICAL MICROBIOLOGY, vol. 30, no. 9, September 1992, pages 2307-2310, XP002038723 WASHINGTON, US cited in the application
- DAVIS ET AL.: "Nucleotide sequence of a HCMV DNA fragment encoding a 67 KD phosphorilated viral protein" JOURNAL OF VIROLOGY, vol. 56, no. 1, 1985, pages 7-11, XP002038724
- BECK ET AL.: "An HCMV reading frame which has similarity with both the V and C regions of the TCR gamma chain" DNA SEQUENCE, vol. 2, 1991, pages 33-38, XP002038725

### SEQUENCE LISTING

(1) GENERAL INFORMATION:
   (i) APPLICANT:
      (A) NAME: Akzo Nobel N.V.
      (B) STREET: Velperweg 76
      (C) CITY: Arnhem
      (E) COUNTRY: The Netherlands
      (F) POSTAL CODE (ZIP): 6824 BM
   (ii) TITLE OF INVENTION: Improved oligonucleotides for the amplification and detection of CMV nucleic acid
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.30 (EPO)
(2) INFORMATION FOR SEQ ID NO: 1:
   (i) SEQUENCE CHARACTERISTICS:
      (A) LENGTH: 17 base pairs
      (B) TYPE: nucleic acid
      (C) STRANDEDNESS: single
      (D) TOPOLOGY: unknown
   (ii) MOLECULE TYPE: cDNA to mRNA
   (vi) ORIGINAL SOURCE:
      (A) ORGANISM: Cytomegalovirus
   (xi) SEQUENCE DESCRIPTION: SEQ ID NO: 1:
      GGGTCGATTC AGACTGA 17
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: cDNA to mRNA
   (vi) ORIGINAL SOURCE:
      (A) ORGANISM: Cytomegalovirus
   (xi) SEQUENCE DESCRIPTION: SEQ ID NO: 2:
      CTGGAGATAT ATGTTGACCA 20
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (vi) ORIGINAL SOURCE:
      (A) ORGANISM: Cytomegalovirus
   (xi) SEQUENCE DESCRIPTION: SEQ ID NO: 3:
      GGATTCGGAC TTTCCGTTCG A 21
(2) INFORMATION FOR SEQ ID NO: 4:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: cDNA to mRNA
   (vi) ORIGINAL SOURCE:
      (A) ORGANISM: Cytomegalovirus
   (xi) SEQUENCE DESCRIPTION: SEQ ID NO: 4:
      CCAAAAAGCT AGCCGTCACG 20

## Description

The present invention is concerned with oligonucleotides that can be used as in the amplification detection of human Cytomegalovirus (HCMV) mRNA. Furthermore a method for the diagnosis of HCMV disease is provided.

Human Cytomegalovirus is an ubiquitous Herpes-type virus, having a double stranded DNA genome of about 240,000 nucleotides in length that infects 40-80% of humans before puberty. A prominent feature common to all herpesviruses is their establishment of lifelong persistence after infection and their ability to cause recurrent infection after reactivation (Stevens, J.G.. Microbiol. Rev. 53, 318-332., 1989). HCMV also becomes latent after primary infection which often occurs without clinical symptoms. Even recurrent infection in most cases goes asymptomatic or leads to only mild disease in the immunocompetent host. However, in congenitally infected infants and immunocompromised patients, such as allograft recipients (Meyers, J.D., et al., J. Infect. Dis. 153, 478-488., 1986) or AIDS patients (Drew, W.L. J Infect. Dis 158, 449-456., 1988; Drew, W.L. Clin. Infect. Dis 14, 608-615., 1992), where the fine balance between the immune system and the latently existing virus is disturbed, HCMV may cause severe and sometimes life-threatening disease, including retinitis, gastrointestinal disorders, and encephalitis (Drew, 1992). Early administration of antiviral drugs like ganciclovir and foscarnet can have significant beneficial effects on the prognosis of a patient (Jahn, G. et al., Intervirology 35, 60-72., 1993; Schmidt, G.M. et al., N. Engl. J Med. 324, 1005-1011., 1991). Therefore, with the availability of clinically effective antiviral therapy, early and sensitive diagnosis is of significant importance.

CMV specific antibodies, in particular IgM antibodies, can be used as a marker for CMV infection, but are of limited value when it comes to discrimination between latent and active infections. Most viral detection methods currently employed do not unambiguously allow for prediction of whether a given infection will be symptomatic. Furthermore serological methods are indirect and often lack sensitivity. Viral culture is a more direct diagnostic parameter for CMV viremia. Although CMV culture from blood cells appeared to be indicative for an active CMV infection, the method does not enable rapid diagnosis and is technically difficult. Moreover, viral culture does not necessarily correspond to HCMV disease. A reliable relation between virus isolation from peripheral leukocytes and the appearance of clinical symptoms may not exist in some immunosuppressed patients (Delgado, R. et al., J Clin. Microbiol. 30, 1876-1878., 1992). Also urinary or pharyngeal shedding of the virus frequently occurs without clinical symptoms and organ involvement. Amplification of HCMV DNA in peripheral leukocytes by polymerase chain reaction (PCR), although a very sensitive technique for CMV viremia, is not usable as a marker of clinically symptomatic HCMV infection either. Due to the high sensitivity of enzymatic amplification, occasionally HCMV DNA was detectable in peripheral leukocytes without HCMV-related disease. Latent viral genomes may be detected by this technique or a patient may remain HCMV-DNA positive over a prolonged period of time after the disease has resolved (Jahn, G. et al., 1993; Zipeto, D. et al., J Clin. Microbiol. 30, 527-530., 1992; Delgado et al., 1992).

At the moment, the method of choice for the early diagnosis of acute symptomatic HCMV infection is the antigenemia assay based on immunological detection of the structural protein pp65 by using specific antibodies (Storch, G.A., et al., J. Clin. Microbiol. 32, 997-1003., 1994; Gerna, G. et al., J. Infect. Dis. 164, 488-498., 1991; Gerna, G., et al., J Clin. Microbiol. 30, 1232-1237.98., 1992). However, a matter of concern employing this method is its sensitivity. The number of pp65-positive cells in the early course of infection may be very low. Furthermore, in expressing cells stability of the pp65 antigen appeared to be limited (Chou, S., Curr. Opin. Infect. Dis. 5, 427-432., 1991) and sensitivity can be reduced due to the application of monoclonal antibodies rather than a pool of anti-pp65 antibodies that would recognize different epitopes of the protein.

Since viral replication requires transcription of mRNA species, the use of HCMV mRNA detection as a marker for active CMV infection was investigated (Bitsch, A. et al., J Infect. Dis 167, 740-743, 1993.

Recently, HCMV infections were examined on the transcript level using RNA amplification (Bitsch, A. et al., 1993; Meyer, T. et al., Mol. Cell Probes. 8, 261-271., 1994; Gerna, G., et al., J Clin. Microbiol. 30, 1232-1237.98, 1993; Gerna, G., et al., J Clin. Microbiol. 30, 1232-1237.98, 1992). In principle, like detection of viral antigens, analysis of viral transcripts expressed in association with viral replication should allow reliable diagnosis of symptomatic infections.

More recently, the detection of certain mRNA's of HCMV i.e. based on IEA (immediate early antigen) and the matrix tegument protein pp67 mRNA have been described in WO 96/06191. However, the oligonucleotides, when used in the amplification and as probes in the detection of the HCMV pp67 mRNA as disclosed in said patent-application have several disadvantages when compared with the oligonucleotides according to the present invention. In comparison studies it became clear that the novel oligonucleotides of the present invention has several advantages over the oligonucleotides as disclosed in WO 96/06191. These studies are presented in the experimental part of the description.

The sensitivity and reliability (robustness) of CMV mRNA detection is greatly dependent on the selection of the oligonucleotides used in the amplifcation, since there is sequence variation among strains of CMV potentially in every region of the genome. Ideally, primer selection should be based on knowledge of interstrain variability in candidate primer sequences and the consequences of mismatching at primer sites. (Chou S., J. of Clin. Microbiol., 2307-2310, 1992).

Therefore, the need exists for suitable oligonucleotides including nucleic acid sequences that can be used in the amplification and subsequent detection of all strain variants of CMV.

The present invention is related to the detection of a certain late HCMV mRNA and provides oligonucleotides suitable for use in the amplification and subsequent detection of this mRNA. The binding sites of the oligonucleotides according to the present invention are located in the matrix tegument protein pp67 encoding gene sequence (UL65), expressed during the late phase of CMV infection.

Oligonucleotides according to the present invention are 10-35 nucleotides in length and comprise, at least a fragment of 10 nucleotides, of sequence:
5'-CTGGAGATATATGTTGACCA-3' [SEQ. ID.No.: 2],
or its complementary sequence.

A preferred embodiment of the present invention is directed to an oligonucleotide linked to a promoter sequence.

A preferred embodiment of the present invention is directed to the following oligonucleotide sequences:
5'-aattctaatacgactcactatagggagaGGGTCGATTCAGACTGA-3' in combination with 5'-CTGGAGATATATGTTGACCA-3'.

The T7 promoter sequence is shown, but may be replaced by any other suitable promoter sequence.

As already indicated above, and will be presented in the experimental part of the description, both the sensitivity and reliability of CMV mRNA detection is greatly improved using the oligonucleotides according to the present invention when compared to known oligonucleotides used in this art.

The term "oligonucleotide" as used herein refers to a molecule comprised of two or more deoxyribonucleotides or ribonucleotides such as primers and probes.

The term "primer" as used herein refers to an oligonucleotide either naturally occurring (e.g. as a restriction fragment) or produced synthetically, which is capable of acting as a point of initiation of synthesis of a primer extension product which is complementary to a nucleic acid strand (template or target sequence) when placed under suitable conditions (e.g. buffer, salt, temperature and pH) in the presence of nucleotides and an agent for nucleic acid polymerization, such as DNA dependent or RNA dependent polymerase. A primer must be sufficiently long to prime the synthesis of extension products in the presence of an agent for polymerization. A typical primer contains at least about 10 nucleotides in length of a sequence substantially complementary (P1) or homologous (P2) to the target sequence, but somewhat longer primers are preferred. Usually primers contain about 15-26 nucleotides but longer primers may also be employed.

Normally a set of primers will consist of at least two primers, one 'upstream' and one 'downstream' primer which together define the amplificate (the sequence that will be amplified using said primers).

The oligonucleotides according to the invention may also be linked to a promoter sequence. The term "promoter sequence" defines a region of a nucleic acid sequence that is specifically recognized by an RNA polymerase that binds to a recognized sequence and initiates the process of transcription by which an RNA transcript is produced. In principle any promoter sequence may be employed for which there is a known and available polymerase that is capable of recognizing the initiation sequence. Known and useful promoters are those that are recognized by certain bacteriophage RNA polymerases such as bacteriophage T3, T7 or SP6.

It is understood that oligonucleotides consisting of the sequences of the present invention may contain minor deletions, additions and/or substitutions of nucleic acid bases, to the extent that such alterations do not negatively affect the yield or product obtained to a significant degree.

An oligonucleotide sequence used as detection-probe may be labeled with a detectable moiety. Various labeling moieties are known in the art. Said moiety may, for example, either be a radioactive compound, a detectable enzyme (e.g. horse radish peroxidase (HRP)) or any other moiety capable of generating a detectable signal such as a colorimetric, fluorescent, chemiluminescent or electrochemiluminescent signal. Preferred analysis systems wherein said labels are used are electrochemiluminescence (ECL) based analysis or enzyme linked gel assay (ELGA) based analysis.

Another preferred embodiment of the present invention is directed to a method for the diagnosis of symptomatic CMV disease, wherein the presence of pp67 mRNA encoding a late structural protein of the human Cytomegalovirus in a blood sample of an individual, suspected of carrying said disease, is detected, said method comprising the following steps:
- amplifying a target sequence within said mRNA using oligonucleotides according to the invention and suitable amplification reagents,
- reacting the sample, optionally containing amplified nucleic acid, with an oligonucleotide according to the present invention as a detection-probe,
- detecting hybrids formed between the amplified sequence and the probe.

Various techniques for amplifying nucleic acid are known in the art. One example of a technique for the amplification of a DNA target segment is the so-called "polymerase chain reaction" (PCR). With the PCR technique the copy number of a particular target segment is increased exponentially with a number of cycles. A pair of primers is used and in each cycle a DNA primer is annealed to the 3' side of each of the two strands of the double stranded DNA-target sequence. The primers are extended with a DNA polymerase in the presence of the various mononucleotides to generate double stranded DNA again. The strands of the double stranded DNA are separated from each other by thermal denaturation and each strand serves as a template for primer annealing and subsequent elongation in a following cycle. The PCR method has been described in Saiki et al., Science 230, 135, 1985 and in European Patents no. EP 200362 and EP 201184.

Another technique for the amplification of nucleic acid is the so-called transcription based amplification system (TAS). The TAS method is described in International Patent Appl. no. WO 88/10315. Transcription based amplification techniques usually comprise treating target nucleic acid with two oligonucleotides one of which comprises a promoter sequence, to generate a template including a functional promoter. Multiple copies of RNA are transcribed form said template and can serve as a basis for further amplification.

An isothermal continuous transcription based amplification method is the so-called NASBA process ("NASBA") as described in European Patent no. EP 329822. NASBA includes the use of T7 RNA polymerase to transcribe multiple copies of RNA from a template including a T7 promoter.

For RNA amplification (as with the method according to the invention), the NASBA technology, or another transcription based amplification technique, is a preferred technology.

If RT-PCR is used for the detection of viral transcripts differentiation of mRNA- and DNA-derived PCR products is necessary. For spliced transcripts, like the IEA mRNA, the exon-intron structure can be used. However, mRNA species encoding the late structural proteins are almost exclusively encoded by unspliced transcripts. DNAse treatment prior to RT-PCR can be employed (Bitsch, A. et al., J Infect. Dis 167, 740-743., 1993; Meyer, T. et al., Mol. Cell Probes. 8, 261-271., 1994), but sometimes fails to remove contaminating DNA sufficiently (Bitsch, A. et al., 1993).

In contrast to RT-PCR, NASBA, which is based on RNA transcription by T7 RNA polymerase (Kievits et al., 1991; Compton, 1991), does not need differentiation between RNA- and DNA-derived amplification products since it only uses RNA as its principal target. NASBA enables specific amplification of RNA targets even in a background of DNA. Especially for unspliced targets like almost all late HCMV gene transcripts, this method is beneficial as it circumvents DNAse treatment which occasionally might be inefficient (Bitsch, A. et al., 1993).

This method was used for the analysis of CMV transcripts in whole blood samples from HIV-infected individuals.

Test kits for the detection of CMV in clinical samples are also part of the present invention. A test kit according to the invention may comprise a pair of oligonucleotides according to the invention and a probe comprising an oligonucleotide according to the invention. Such a test kit may additionally comprise suitable amplification reagents such as DNA and or RNA polymerases and mononucleotides. Test kits that can be used with the method according to the invention may comprise the oligonucleotides according to the invention for the amplification and subsequent detection of pp67 mRNA.

The invention is further exemplified by the following examples.

### EXAMPLES:

### Example 1: Analytical sensitivity of CMV-pp67 oligonucleoitdes in amplification and detection.

### 1.1. Materials and methods

### 1.1.1. In vitro RNA

RNA with a length of 1125 nucleotides encompassing 338 nucleotides of the CMV mRNA encoding the pp67 matrix tegument protein was synthesized in vitro from a cloned fragment of the corresponding gene by T7 RNA polymerase-based transcription. Prior to transcription, plasmid DNA was linearized by Bam Hl digestion, the unique restriction site of which is located about 800 base pairs (bp) downstream of the CMV insert. The digested DNA was purified by phenol extraction and concentrated by ethanol precipitation. Transcription from the linearized plasmid DNA was performed in transcription buffer [40 mM Tris-Hydrochloric acid (pH 7.5); 6 mM Magnesium chloride; 2 mM Spermidine; 10 mM Sodium chloride] supplemented with 0.5 mM of each rNTP, 10 mM Dithiothreitol (DTT), 1 unit per µl RNA Guard (Pharmacia), and about 500 units T7 RNA Polymerase (Pharmacia). After 4 hours incubation at 37°C. DNase I (Boehringer) was added to a final concentration of 0.1 unit per µl and the reaction mixture incubated at 37°C for an additional 30 minutes. Subsequently, in vitro generated RNA was purified from the reaction mixture using a RNeasy RNA Purification kit (Qiagen). Finally, the concentration of the in vitro RNAs was determined by OD (260 nm) measurement and appropriate serial dilutions in water were stored at -70°C.

### 1.1.2. Oligonucleotides used in amplification and as probes

Sequences and polarity of the oligonucleotides used in the amplification and of the probes used for specific detection, are shown in Table 1.

All oligonucleotides were synthesized on a PCR-MATE 391 DNA synthesizer (Applied Biosystems) using phosphoramidite biochemistry. Oligonucleotides for ELGA detection (see below) were synthesized with a 5'-amino link (Aminolink 2; Applied Biosystems) for subsequent coupling of Horse Radish Peroxidase (HRP).

Oligonucleotides used in amplification were purified by electrophoretically separating the crude oligonucleotide solutions over a 20% polyacrylamide/7M Urea slabgel and subsequent elution of the full-length oligonucleotide from the corresponding gel band. After elution from the gel slices and concentration by ethanol precipitation, oligonucleotides were dissolved in Milli-Q water and concentrations determined by OD(260 nm) measurement.

For ELGA detection, oligonucleotide probe CMV-pp67 HRP1 was conjugated with HRP (Boehringer) by coupling the enzyme to the amino link of the oligonucleotide using the crosslinking reagents SDPD (Pharmacia) and EMCS (Fluka). Unbound HRP was removed over a Qiagen Tip-100 column (Qiagen). The HRP-labeled oligonucleotide was purified by polyacrylamide gel electrophoresis and subsequent elution of the HRP-oligonucleotide from the gel slices by overnight incubation in water. The amount of HRP-conjugated oligonucleotide was calculated from OD(260nm) and OD(400 nm) measurement. The solutions were stored at -70°C.

For ECL detection, oligonucleotide probe CMV-pp67-ECL (table 1) was conjugated with the ECL label by incubating the amino link oligonucleotide with TAG NHS-Ester (lgen). Unbound label was removed by passing the reaction mixture over a Qiagen Tip-100 column (Qiagen). The amount of ECL-labelled oligonucleotide was calculated from OD (260 nm) and OD (460 nm) measurement. The solution was stored at -70°C and used without further purification.

### 1.1.3. NASBA amplification

RNA amplifications were performed using the NASBA amplification technology. To set up a NASBA amplification reaction, a premix was generated by mixing 4 µl of 5x reaction buffer [200 mM Tris-Hydrochloric acid (pH 8.5), 350 mM Potassium chloride, 60 mM Magnesium chloride, 25 mM DTT, 5 mM of each dNTP, 10 mM of ATP, CTP and UTP, 7.5 mM of GTP and 2.5 mM of ITP] with 2 µl sugar solution [15% (w/v) Sucrose, 5% (w/v) Mannitol, 5% (w/v) Dextrane T40] and 4 µl of a mix containing 1 µM of each oligonucleotide to be used in the amplificaton in 75% DMSO. Of this premix, 10 µl was added to 5 µl nucleic acid solution and incubated during 5 minutes at 65°C. Subsequently, the reaction tubes were incubated at 41 °C during 5 minutes before 5 µl enzyme mix [32 units T7 RNA polymerase; 6.4 units AMV reverse transcriptase; 0.08 unit RNase H; 2.1 µg BSA; 20 mM DTT; 1.5 M Sorbitol] was added. After the final addition, tubes were mixed by gentle tapping, centrifuged, and incubated at 41 °C during 90 minutes. Reactions were stopped by placing them at -20°C.

### 1.1.4. Analysis of NASBA-amplified reaction products by ELGA

For the analysis of NASBA reaction products a non-radio-active enzyme linked gel assay (ELGA) based on liquid hybridization was used. Hybridization of amplification product to a specific HRP-labelled oligonucleotide probe was performed by mixing 3 µl of a NASBA amplification reaction with 1 µl 6xSSC, 1 µl concentrated loading buffer [25% (v/v) Glycerol; 10 mM Sodium phosphate buffer (pH 7.0); 0.05% Bromophenol blue; 0.01 % Xylene cyanol], and 1 µl HRP-labelled oligonucleotide CMV-pp67 HRP1 (table 1) stock solution, followed by incubation at 45°C during 15 minutes. After hybridization, half of the reaction mixture was directly applied onto a 7% polyacrylamide gel supplemented with 0.04% (w/v) dextrane sulphate. After separation of bound and unbound HRP-labeled oligonucleotide by electrophoresis, the probe was visualized in the gel by direct staining with 50 ml substrate solution [125µg 3,3',5,5'-tetramethylbenzidine per ml; 0.003 % Hydrogen peroxide; 100mM Sodium citrate buffer (pH 5.2)] for about 10 minutes at room temperature. Finally, the gel was fixed by overnight incubation in a 50% (v/v) methanol solution and air dried.

### 1.2. Results

### 1.2.1. Analytical sensitivity of CMV-pp67 oligonucleotides when used in NASBA

To determine the analytical sensitivity of the improved CMV-pp67 NASBA pair of oligonucleotides according to the present invention (CMV-pp67-5) and to compare this sensitivity with the analytical sensitivity of the known (WO 96/06191) CMV-pp67 pair (CMV-pp67-4) (table 1), a dilution series of in vitro generated RNA encompassing the target sequence of both pairs was prepared. The individual samples of this dilution series contained 10.000, 1.000, 100 and 10 molecules of in vitro generated RNA, respectively. In several independent experiments NASBA amplification of this dilution series was performed with these two CMV-pp67 NASBA pairs of oligonucleotides (Table 1). A typical example is shown in Table 2.

Direct comparison of the pairs showed that in this example the lowest amount of in vitro RNA molecules that could be detected with CMV-pp67-4 was 1.000 molecules, whereas with the improved combination CMV-pp67-5 also 100 molecules of input RNA revealed a positive NASBA result.

Table 3 summarizes the results of five independent experiments. For each pair, the lowest in vitro RNA amount still giving a positive NASBA result in a particular experiment, is shown.

The pairs that were analyzed, have a comparable analytical sensitivity in NASBA, since with both combinations presence of 100 molecules of in vitro generated RNA in a sample can be demonstrated upon NASBA amplification. However, in three out of five analyses the CMV-pp67-4 combination appeared to have a lower detection limit of 1.000 molecules of in vitro generated RNA, whereas CMV-pp67-5 consistently revealed a positive NASBA result for 100 molecules of input RNA in all analyses that were performed (Table 3).

Therefore, the CMV-pp67-5 combination was regarded as more robust for NASBA amplification. It was anticipated that this advantageous aspect of this novel pair of oligonucleotides does not only hold true for the detection of in vitro generated RNA, but also for detection of the genuine CMV-pp67 protein encoding mRNA as encountered in clinical specimens of CMV infected individuals.

### Example 2: Detection of CMV-pp67 protein encoding mRNA in clinical samples.

### 2.1, Materials and methods

### 2.1.1. Clinical specimens

495 samples were obtained from a cohort of 134 AIDS patients. These ethylene diaminotetraacetic acid (EDTA) anticoagulated whole blood samples submitted consecutively as received by the laboratory, were mixed with nine volumes of lysisbuffer [50 mM Tris-Hydrochloric acid (pH 6.4); 20 mM EDTA; 1.3% (w/v) Triton X-100; 5.25 M Guanidinium thiocyanate] and stored at -70°C until use.

### 2.1.2. Oligonucleotides

Sequences and polarity of the oligonucleotides used in amplification and detection , are shown in table 1. For further details is referred to the paragraph 1.1.2. in the Materials and Methods section of Example 1.

### 2.1.3. Nucleic acid isolation

From the anticoagulant-treated blood specimens total nucleic acid was isolated using guanidinium thiocyanate-mediated cell lysis and adsorption of nucleic acid to silica particles (Boom et al., J. of Clin. Microbiol. 28, 495-503, 1990).

Whole blood samples in Lysis buffer were thawed and from each sample 1 ml (equivalent to 100 µl whole blood) was transferred into an Eppendorf tube. Subsequently, 50 µl of Hydrochloric acid-activated silicum dioxide particles [size-selected suspension of 1 mg/ml in 0.1 M Hydrochloric acid (Sigma); see ref. Boom et al., 1990] were added and the suspension was incubated during 10 minutes at room temperature with regular vortexing. Nucleic acid bound to the silica was spun down by centrifugation. Pelleted silica particles were washed twice with 1 ml GuSCN wash buffer [50 mM Tris-Hydrochloric acid (pH 6.4); 5.25 M Guanidinium thiocyanate], followed by two washing steps with 1 ml 70% ethanol and a single washing step with 1 ml acetone. After each washing step, the suspension was briefly centrifuged and the silica pellet was resuspended in the next washing solution by thorough mixing. After removal of the acetone, the silica particles were dried by incubation at 56°C in a heating block during 10 minutes. Nucleic acid was eluted from the silica particles by incubation in 50 µl Tris-buffered elution medium (pH 7.5) at 56°C during 10 minutes. Finally, the silica particles were spun down again and the supernatant was carefully pipetted into fresh reaction tubes avoiding any carry-over of silica. Extracted nucleic acid samples were stored at -70°C until use.

### 2.1.4. NASBA amplification

For amplification, a so-called accusphere containing all the ingredients necessary for a NASBA reaction (see "NASBA amplification" paragraph in the Materials and Methods section of Example 1) in a lyophilized form, was reconstituted in a Tris-HCl (pH 8.5) buffered solution of 30% (v/v) DMSO. Subsequently, for each sample to be analysed, 5 µl nucleic acid solution and 10 µl of the oligonucleotide solution were added to a test tube. The resulting mixtures were heated at 65°C for 5 minutes, after which the tubes were placed at 41 °C. After 5 minutes incubation at 41°C, 5 µl of enzyme solution containing 32 units T7 RNA polymerase, 6.4 units of AMV reverse transcriptase and 0.08 unit RNase H were added and the contents of the tube were mixed by gentle tapping. The reactions were incubated at 41°C for 90 minutes in a water bath. Reactions were stopped by placing them at -20°C.

### 2.1.5. Analysis of NASBA amplified reaction products by ECL detection

For electrochemiluminescence (ECL) based analysis, detection reagents were prepared by vortexing a capture probe solution, containing biotinylated capture probe CMV-pp67 CAP (table 1) immobilized on streptavidin-coated paramagnetic beads (Dynal), until an opaque solution was formed and subsequently mixing 10 µl of this suspension (containing 3.4x10⁵ beads loaded with capture probe) and 10 µl of a CMV-pp67-ECL (table 1) probe solution (containing 3.4x10¹¹ molecules of ECL labeled probe) into fresh reaction tubes. To these mixtures, 5 µl of a two-fold diluted NASBA reaction was added and incubated during 30 minutes at 41 °C. During hybridisation, the tubes were mixed every 10 minutes. Subsequently, 300 µl NASBA QR System Assay buffer (Organon Teknika) were added to each hybridisation tube and the tubes were positioned in a NASBA QR System for automated reading of ECL signals.

### 2.2. Results

### 2.2.1. Detection of CMV-pp67 mRNA in clinical samples with two different pairs of oligonucleotides.

Whole blood samples from patients clinically at risk of infection with CMV were analysed for the presence of CMV mRNA encoding the matrix tegument protein pp67 (CMV-pp67 mRNA) with the two distinct pairs of oligonucleotides (table 1) suited for NASBA amplification.

In total, 495 samples from AIDS patients, previously analysed by NASBA amplification with combination CMV-pp67-4 (table 1), now were reanalyzed under similar NASBA conditions with the improved pair CMV-pp67-5 which is the object of the present invention. In table 4, the results obtained with either of the pairs are shown and compared to each other.

Although from earlier experiments no (statistically) significant difference in the analytical sensitivity of these sets of oligonucleotides could be determined (due to the fact that only a few samples were tested; see Example 1), the improved CMV-pp67-5 set appeared to be much better for the detection of CMV-pp67 mRNA by NASBA in nucleic acid solutions extracted from whole blood samples. Due to the amount of samples (n=495), this difference is also statistical significant.

In 65 samples, the improved pair CMV-pp67-5 revealed a positive NASBA result, whereas NASBA reactions performed with the CMV-pp67-4 pair were negative. In only 9 cases (of which 5 samples are difficult to interpret) it was the other way around.

In conclusion, apart from being more robust as the CMV-pp67-4 set (see Example 1) the novel and improved pair of oligonucleotides according to the present invention (CMV-pp67-5) also appears to be superior in the detection of CMV-pp67 mRNA in clinical samples.

### Example 3: Detection of CMV-pp67 protein encoding mRNA in clinical samples

### 3.1. Materials and Methods

### 3.1.1. Clinical specimens

Eighteen ethylene diaminotetraacetic acid (EDTA) anticoagulated whole blood specimens were obtained from a cohort of AIDS patients. Within several hours after bleeding, the samples were mixed with nine volumes of lysis buffer (see paragraph 2.1.1) and stored at -70 °C until use.

Ten heparin anticoagulated whole blood specimens were obtained from kidney transplant recipients taken at time points when the patients were suspected to suffer from a CMV-infection. As for the AIDS patients specimens, also these samples were added to lysis buffer on the day of bleeding and stored at -70 °C until use.

### 3.1.2. Primers and probes

Sequences and polarity of the primers and probes used for specific detection are shown in Table 1. For further details is referred to paragraph 1.1.2 in the Materials and Methods section of Example 1.

### 3.1.3. Nucleic acid isolation

Total nucleic acid was isolated from the anticoagulant treated blood specimen in lysis buffer essentially as described in the "Nucleic acid isolation" paragraph in the Materials and Methods section of Example 2. Extracted nucleic acid samples were analysed by NASBA amplification without prior storage at -70 °C.

### 3.1.4. NASBA amplification

RNA amplifications were performed essentially as described in the "NASBA amplification" paragraph in the Materials and Methods section of Example 1. Each nucleic acid extract was analysed in duplicate with both primer pair CMV-pp67-4 and CMV-pp67-5 (see Table 1).

### 3.1.5. Analysis of NASBA amplified reaction products by ELGA

For the analysis of NASBA reaction products by enzyme-linked gel assay (ELGA) is referred to paragraph 1.1.4 in the Materials and Methods section of Example 1. However, the hybridisation temperature used here was 41°C instead of 45°C as in Example 1.

### 3.1.6. Analysis of NASBA amplified reaction products by ECL detection

Electrochemiluminescence (ECL) based analysis was performed essentially as described in paragraph 2.1.5 in the Materials and Methods section of Example 2. As for ELGA detection (paragraph 3.1.5) a hybridisation temperature of 41°C as used.

### 3.2. Results

### 3.2.1 Detection of CMV-pp67 mRNA amplicons generated with two different primer pairs

In the previous example (Example 2), RNA amplicons generated by NASBA amplification employing primer pair CMV-pp67-4 (Table 1) were analysed by ELGA detection, whereas CMV-pp67 RNA amplified from the same clinical specimens with primer pair CMV-pp67-5 was detected by ECL-based analysis. For a more direct comparison of these NASBA primer pairs for CMV-pp67 mRNA, in this example nucleic acid extracts were amplified with either primer pair and subsequently analysed by the same detection method. For each amplification reaction, ELGA results as well as ECL results were generated.

Total nucleic acid was extracted from eighteen whole blood specimens of AIDS patients clinically at risk of infection with CMV. Subsequently, CMV-pp67 mRNA was amplified in duplicate from these extracts by NASBA amplification using either primer pair CMV-pp67-4 or CMV-pp67-5. Finally, amplicons generated in each of the individual amplification reactions were analysed by ELGA using the horse radish peroxidase (HRP)-labelled probe CMV-pp67 HRP-1 (Table 1) and by ECL-based detection with probe CMV-pp67 ECL (Table 1) in combination with CMV-pp67 CAP, which is a biotynilated version of the ELGA-probe oligonucleotide. In a similar way, ten clinical specimens of kidney transplant patients were analysed and results are summarised in Table 5. Comparing the ELGA results obtained with either primer pair, for 10 individual amplification reactions primer pair CMV-pp67-5 revealed a positive result, whereas the corresponding amplification of the same nucleic acid extract with primer pair CMV-pp67-4 was negative (Table 5, samples 4, 6, 10, 12, 13, 17, 18, 26 (2 reactions) and 27). In only 4 cases (samples 9 (2 reactions), 10, 28) it was vice versa.

Comparison of the ECL-data revealed a similar result: 16 discordant results, 11 of which concerning a positive result with primer pair CMV-pp67-5 versus a negative result with primer pair CMV-pp67-4 (samples 4, 6, 7, 12 (2 reactions), 13, 15, 18, 26 and 27 (2 reactions). For 5 reactions it was the other way around (samples 5, 7, 9, 10 and 18).

Comparison on the sample level and assuming a specimen positive for CMV-pp67 mRNA if one or both individual amplification-reactions are positive, would have revealed eight specimens positive for this CMV mRNA with primer pair CMV-pp67-5 that would have been negative with primer pair CMV-pp67-4 when using ELGA-detection (samples 4, 6, 12, 13, 17, 18, 26 and 27) and one specimen negative with primer pair CMV-pp67-5 that is positive with primer pair CMV-pp67-4 (sample 9). Using ECL-detection, five samples would not have been recognized as CMV-pp67 mRNA positive by primer pair CMV-pp67-4 and again only a single sample would have been missed by primer pair CMV-pp67-5.

In conclusion, CMV-pp67-5 is a more sensitive primer pair for NASBA amplification of CMV-pp67 mRNA than CMV-pp67-4 irrespective of the detection method that is being employed (be it ELGA or ECL-based detection) to analyse the amplicons generated with either primer pair.

**Table 1: Oligonucleotides used in NASBA amplification and detection of CMV-pp67 mRNA**

| pair | Oligonucleotides | SEQ.ID.No.: | Sequence |
|---|---|---|---|
| CMV- | CMV-pp67 P1.2 | | 5'-aattctaatacgactcactatagggagaGGGTCGATTCAGACTGA-3' |
| pp67-4 | CMV-pp67 P2.1 | | 5'-GACCTGATATCCCTCCATATA-3' |
| | CMV-pp67 HRP1 | | 5'-GGATTCGGACTTTCCGTTCGA-3' |
| CMV- | CMV-pp67 P1.2 | SEQ.ID.No.: 1 | 5'-aattctaatacgactcactatagggagaGGGTCGATTCAGACTGA-3' |
| pp67-5 | CMV-pp67 P2.4 | SEQ.ID.No.: 2 | 5'-CTGGAGATATATGTTGACCA-3' |
| | CMV-pp67-CAP | SEQ.ID.No.:3 | 5'-BIO-GGATTCGGACTTTCCGTTCGA-3' |
| | CMV-pp67-ECL | SEQ.ID.No.:4 | 5'-ECL-CCAAAAAGCTAGCCGTCACG-3' |

| | | | |
|---|---|---|---|
| T7 promoter sequence is given in small characters. | | | |

**Table 2: Analytical sensitivity of two CMV-pp67 pairs of oligonucleotides when used in NASBA.**

| | ELGA result | |
|---|---|---|
| Input RNA | CMV-pp67-4 | CMV-pp67-5 |
| 10⁴ | + | + |
| 10³ | + | + |
| 10² | - | + |
| 10¹ | - | - |

**Table 3: Comparison of two CMV-pp67 pairs of oligonucleotides used in NASBA.**

| | Lower detection limit* | |
|---|---|---|
| Experiment | CMV-pp67-4 | CMV-pp67-5 |
| 1 | 10³ | 10² |
| 2 | 10² | 10² |
| 3 | 10² | 10² |
| 4 | 10² | 10² |
| 5 | 10³ | 10² |

| | | |
|---|---|---|
| * Lower detection limit: lowest amount of input RNA that still reveals a positive NASBA result | | |

**Table 4: Analysis of CMV-pp67 mRNA oligonucleotides in amplifications performed on clinical specimens (n=495)**

| | | novel CMV-pp67-5 oligo's | |
|---|---|---|---|
| | | positive | negative |
| (known) CMV-pp67-4 oligo's | positive | 53(10)* | 4(5)* |
| | negative | 65 | 358 |

| | | | |
|---|---|---|---|
| * between brackets the number of weakly positive samples obtained with the CMV-pp67-4 pair is given | | | |

**Table 5: Analysis of clinical whole blood specimens by NASBA with two different CMV-pp67 mRNA primer pairs.**

| Sample No. | Sample identification | Sample source | CMV-pp67-5 | | CMV-pp67-4 | |
|---|---|---|---|---|---|---|
| | | | ELGA | ECL | ELGA | ECL |
| 1 | 93.1 | AIDS patient | +/+ | +/+ | +/+ | +/+ |
| 2 | 93.2 | " | +/+ | +/+ | +/+ | +/+ |
| 3 | 93.3 | " | +/+ | +/+ | +/+ | +/+ |
| 4 | 93.4 | " | +/- | +/- | -/- | -/- |
| 5 | 94.5 | " | -/- | -/- | -/- | +/- |
| 6 | 94.6 | " | +/- | +/+ | -/- | +/- |
| 7 | 94.7 | " | -/- | +/- | -/- | -/+ |
| 8 | 95.18 | " | (+)/+ | +/+ | +/(+) | +/+ |
| 9 | 95.19 | " | -/- | -/+ | (+)/+ | +/+ |
| 10 | 95.20 | " | +/- | +/- | -/(+) | +/+ |
| 11 | 94.9 | " | +/+ | +/+ | (+)/(+) | +/+ |
| 12 | 94.10 | " | +/- | +/+ | -/- | -/- |
| 13 | 94.11 | " | +/- | +/- | -/- | -/- |
| 14 | 94.12 | " | +/+ | +/+ | +/+ | +/+ |
| 15 | 94.13 | " | -/(+) | -/+ | -/(+) | -/- |
| 16 | 94.14 | " | +/+ | +/+ | +/+ | +/+ |
| 17 | 94.16 | " | -/(+) | -/+ | -/- | -/+ |
| 18 | 95.17 | " | (+)/- | +/- | -/- | -/+ |
| 19 | M2896062-1 | kidney transplant | +/+ | +/+ | +/+ | +/+ |
| 20 | M2896062-2 | " | +/+ | +/+ | +/+ | +/+ |
| 21 | M2896062-3 | " | +/+ | +/+ | +/+ | +/+ |
| 22 | M2896062-4 | " | +/+ | +/+ | (+)/(+) | +/+ |
| 23 | M2896062-5 | " | +/+ | +/+ | +/+ | +/+ |
| 24 | M2896062-6 | " | +/- | +/+ | +/- | +/+ |
| 25 | M2896062-7 | " | +/+ | +/+ | +/+ | +/+ |
| 26 | M2896062-8 | " | +/(+) | +/+ | -/- | -/+ |
| 27 | M2896062-19 | " | +/- | +/+ | -/- | -/- |
| 28 | M2896062-20 | " | +/- | +/+ | +/+ | +/+ |

| | | | | | | |
|---|---|---|---|---|---|---|
| + positive (+) weakly positive - negative | | | | | | |

## Claims

1. Oligonucleotide, corresponding to part of a nucleic acid sequence encoding HCMV pp67, said oligonucleotide being 10-35 nucleotides in length and comprising at least a fragment of 10 nucleotides of a sequence consisting of:
5'-CTG GAG ATA TAT GTT GAC CA-3'[SEQ ID No. 2],
or its complementary sequence.

2. Oligonucleotide according to claim 1 linked to a promoter sequence.

3. Pair of oligonucleotides, for the amplification of a target sequence located within a HCMV pp67 sequence, for use as a set, comprising one oligonucleotide being 10-35 nucleotides in length and comprising at least a fragment of 10 nucleotides of a sequence consisting of
5'-GGG TCG ATT CAG ACT GA-3'[SEQ ID No. 1]
and a second oligonucleotide being 10-35 nucleotides in length and comprising at least a fragment of 10 nucleotides of a sequence consisting
5'-CTG GAG ATA TAT GTT GAC CA-3'[SEQ ID No. 2].

4. Method for the diagnosis of symptomatic CMV disease, **characterized in that** the presence of pp67 mRNA encoding a late structural protein of the human cytomegalovirus in a blood sample of an individual, suspected of carrying said disease, is detected, said method comprising the following steps:
- amplifying a target sequence within said mRNA using a pair of oligonucleotides according to claim 3 and suitable amplification reagents;
- reacting the sample, optionally containing amplified nucleic acid, with an oligonucleotide consisting essentially of the sequence 5'-GGA TTC GGA CTT TCC GTT CGA-3' [SEQ ID No. 3] or 5'-CCA AAA AGC TAG CCG TCA CG-3' [SEQ ID No. 4], provided with a detectable label;
- detecting hybrids formed between the amplified sequence and the probe.

5. Method according to claims 4, wherein the mRNA is amplified, using a transcription based amplification technique.

6. Method according to claim 5, wherein the sequence 5'-GGG TCG ATT CAG ACT GA-3'[SEQ ID No. 1] is linked to a promoter sequence.

7. Method according to claim 5, wherein said amplification technique is NASBA.

8. Use of the pair of oligonucleotides according to claim 3 in a nucleic acid amplification reaction.

9. Test kit for the diagnosis of HCMV disease comprising:
- the pair of oligonucleotides according to claim 3;
- an oligonucleotide comprising a nucleic acid sequence substantially complementary to at least part of the amplified nucleic acid sequence , provided with a detectable label;
- suitable amplification reagents.

10. Test kit according to claim 9, wherein said oligonucleotide that is provided with label is an oligonucleotide consisting essentially of the sequence
5'-GGA TTC GGA CTT TCC GTT CGA-3'[SEQ ID No. 3], or
5'-CC AAA AAG CTA GCC GTC ACG-3' [SEQ ID No. 4].

## Patentansprüche

1. Oligonukleotid, entsprechend einem Teil einer Nukleinsäuresequenz kodierend HCMV pp67, wobei das besagte Oligonukleotid eine Länge von 10-35 Nukleotiden aufweist und umfassend mindestens ein Fragment von 10 Nukleotiden einer Sequenz bestehend aus: 5'-CTG GAG ATA TAT GTT GAC CA -3' [SEQ.ID.Nr.: 2], oder ihrer komplementären Sequenz.

2. Oligonukleotid gemäss Anspruch 1 verbunden mit einer Promotersequenz.

3. Paar von Oligonukleotiden für die Verstärkung einer Targetsequenz angeordnet innerhalb einer HCMV pp67 Sequenz, zur Verwendung als ein Set, umfassend ein Oligonukleotid mit einer Länge von 10-35 Nukleotiden und umfassend mindestens ein Fragment von 10 Nukleotiden einer Sequenz bestehend aus 5'-GGG TCG ATT CAG ACT GA-3' [SEQ.ID.Nr. 1] und ein zweites Oligonukleotid mit einer Länge von 10-35 Nukleotiden und umfassend mindestens ein Fragment von 10 Nukleotiden einer Sequenz bestehend aus 5'-CTG GAG ATA TAT GTT GAC CA-3' [SEQ.ID.Nr. 2].

4. Verfahren für die Diagnose der symptomatischen ZMV-Krankheit (Zytomegalievirus), **dadurch gekennzeichnet, dass** die Gegenwart der pp67 mRNS, kodierend ein spätes Strukturprotein des menschlichen Zytomegalievirus in einer Blutprobe eines Individuums, von dem angenommen wird, dass es diese Krankheit hat, festgestellt wird, wobei das Verfahren die folgenden Schritte umfasst:
- Verstärken einer Targetsequenz innerhalb der besagten mRNS unter Einsatz eines Paars von Oligonukleotiden gemäss Anspruch 3 und geeigneter Verstärkungsreagenzien,
- Reagieren der Probe, optional enthaltend verstärkte Nukleinsäure, mit einem Oligonukleotid im wesentlichen umfassend die Sequenz 5'-GGA TTC GGA CTT TCC GTT CGA-3' [SEQ.ID.Nr.: 3] oder 5'-CCA AAA AGC TAG CCG TCA CG-3' [SEQ.ID.Nr.: 4], versehen mit einer erfassbaren Markierung,
- Erfassen von Hybriden, die zwischen der verstärkten Sequenz und der Probe ausgebildet sind.

5. Verfahren gemäss Anspruch 4, bei dem die mRNS verstärkt wird unter Einsatz einer Transkriptions-basierten Verstärkungstechnik.

6. Verfahren gemäss Anspruch 5, bei dem die Sequenz 5'-GGG TCG ATT CAG ACT GA-3' [SEQ.ID.Nr.1] mit einer Promotersequenz verbunden wird.

7. Verfahren gemäss Anspruch 5, bei dem die besagte Verstärkungstechnik NASBA (Nukleinsäuresequenz basierte Verstärkung) ist.

8. Verwendung eines Paares von Oligonukleotiden gemäss Anspruch 3 in einer Nukleinsäure-Verstärkungsreaktion.

9. Testkit für die Diagnose der HCMV Krankheit (Humane Cytomegalievirus Krankheit) umfassend:
- das Paar von Oligonukleotiden gemäss Anspruch 3,
- ein Oligonukleotid umfassend eine Nukleinsäuresequenz im wesentlichen komplementär zu mindestens einem Teil der verstärkten Nukleinsäuresequenz, versehen mit einer erfassbaren Markierung,
- geeignete Verstärkungreagenzien.

10. Testkit gemäss Anspruch 9, bei dem das Oligonukleotid, das mit einer Markierung versehen ist, ein Oligonukleotid ist, bestehend im wesentlichen aus der Sequenz
5'-GGA TTC GGA CTT TCC GTT CGA-3' [SEQ.ID.Nr.: 3], oder
5'-CC AAA AAG CTA GCC GTC ACG-3' [SEQ.ID.Nr.: 4].

## Revendications

1. Oligonucléotide, correspondant à une partie de séquence d'acide nucléique codant pour HCMV pp67, lesdites oligonucléotides ayant la longueur de 10-35 nucléotides et comprenant au moins un fragment de 10 nucléotides d'une séquence consistant en:
5'-CTG GAG ATA TAT GTT GAC CA-3' [SEQ ID No. 2],
ou sa séquence complémentaire.

2. Oligonucléotide selon la revendication 1 liée à une séquence de promoteur.

3. Paire d'oligonucléotides, pour l'amplification d'une séquence cible située dans une séquence HCMV pp67, pour être utilisée en tant que set comprenant une oligonucléotide ayant la longueur de 10-35 nucléotides et au moins un fragment de 10 nucléotides d'une séquence consistant en:
5'-GGG TCG ATT CAG ACT GA-3' [SEQ ID No. 1],
et comprenant une seconde oligonucléotide ayant la longueur de 10-35 nucléotides et au moins un fragment de 10 nucléotides d'une séquence consistant en:
5'-CTG GAG ATA TAT GTT GAC CA-3' [SEQ ID No. 2],

4. Procédé pour le diagnostic d'une maladie symptomatic CMV, **caractérisé en ce que** est détectée la présence de pp67mRNA codant pour une protéine structurelle tardive d'un cytomegalovirus humain dans un échantillon de sang d'un individu, suspecté de présenter ladite maladie, ledit procédé comprenant les étapes suivantes:
- l'amplification d'une séquence cible par ladite mRNA en utilisant une paire d'oligonucléotides selon la revendication 3 et des réactifs d'amplification appropriés;
- la réaction de l'échantillon, contenant éventuellement de l'acide nucléique amplifié avec une oligonucléotide consistant essentiellement en une séquence
5' - GGA TTC GGA CTT TCC GTT CGA-3' [SEQ ID No. 3],
ou
5' CCA AAA AGC TAG CCG TCA CG-3' [SEQ ID No. 4],
pourvue d'un traceur detectable;
- la détection des hybrides formés entre la séquence amplifiée et la sonde.

5. Procédé selon la revendication 4, dans lequel l' ARNm est amplifiée à l'aide d'une technique d'amplification basée sur la transcription.

6. Procédé selon la revendication 5, dans lequel la séquence
5'-GGG TCG ATT CAG ACT GA-3' [SEQ ID No. 1]
est liée à une séquence de promoteur.

7. Procédé selon la revendication 5, dans laquelle ladite technique d'amplification est la technique dite NASBA.

8. Utilisation d'une paire d'oligonucléotides selon la revendication 3 dans une réaction d'amplification d'un acide nucléique.

9. Ensemble ou kit de tests pour le diagnostic de la maladie HCMV comprenant:
- la paire d'oligonucléotides selon la revendication 3;
- un oligonucléotide comprenant la séquence d'acide nucléique substantiellement complémentaire d'au moins une partie d'une séquence d'acide nucléique amplifiée, présentant un traceur détectable;
- des réactifs d'amplification appropriés.

10. Ensemble ou kit de tests selon la revendication 9, dans lequel ladite oligonucléotide qui est pourvue d'un traceur est une oligonucléotide consistant essentiellement en une séquence
5' - GGA TTC GGA CTT TCC GTT CGA-3' [SEQ ID No. 3],
ou
5' -CC AAA AAG CTA GCC GTC ACG-3' [SEQ ID No. 4].
